(19) Europäisches Patentamt / European Patent Office / Office européen des brevets

(11) **EP 3 801 058 B1**

(12) **FASCICULE DE BREVET EUROPEEN**

(45) Date de publication et mention
de la délivrance du brevet:
**07.08.2024 Bulletin 2024/32**

(21) Numéro de dépôt: **19744758.4**

(22) Date de dépôt: **03.06.2019**

(51) Classification Internationale des Brevets (IPC):
*A23L 29/00* (2016.01)  *A61K 8/27* (2006.01)
*A61K 8/33* (2006.01)  *A61K 8/34* (2006.01)
*A61Q 17/04* (2006.01)  *A61Q 19/08* (2006.01)
*A61K 8/04* (2006.01)  *A61K 8/02* (2006.01)

(52) Classification Coopérative des Brevets (CPC):
**A61K 8/347; A23L 33/10; A61K 8/0241;
A61K 8/27; A61K 8/33; A61Q 17/04; A61Q 19/08;**
A61K 2800/10; A61K 2800/413; A61K 2800/522;
A61K 2800/58; A61K 2800/622

(86) Numéro de dépôt international:
**PCT/FR2019/051304**

(87) Numéro de publication internationale:
**WO 2019/229402 (05.12.2019 Gazette 2019/49)**

(54) **MOLECULE MIMANT UNE ACTIVITE ANTIOXYDANTE**

MOLEKÜLE, DIE EINE ANTIOXIDATIVE AKTIVITÄT NACHAHMEN

MOLECULE MIMICKING ANTI-OXIDANT ACTIVITY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.05.2018 FR 1854741**

(43) Date de publication de la demande:
**14.04.2021 Bulletin 2021/15**

(73) Titulaire: **Bionuclei
13290 Aix-en-Provence (FR)**

(72) Inventeurs:
• **ATHALIN, Han
44000 NANTES (FR)**
• **THOREL, Jean-Noël
75014 PARIS (FR)**

(74) Mandataire: **Cabinet Laurent & Charras
Le Contemporain
50 Chemin de la Bruyère
69574 Dardilly Cedex (FR)**

(56) Documents cités:
WO-A1-2017/037731    CN-A- 108 084 494
FR-A1- 2 708 851    US-A1- 2003 049 287
US-A1- 2009 155 371    US-A1- 2010 284 942

• ARSLANOGLU YASIN ET AL: "Synthesis,
electrochemical and photophysical studies of
axially substituted quarternizable titanyl
phthalocyanines", DYES AND PIGMENTS,
ELSEVIER APPLIED SCIENCE PUBLISHERS.
BARKING, GB, vol. 97, no. 2, 4 January 2013
(2013-01-04), pages 340 - 346, XP028980342,
ISSN: 0143-7208, DOI:
10.1016/J.DYEPIG.2012.12.022
• PRIYANKA PRATHIPATI ET AL: "Development of
novel HDL-mimicking [alpha]-tocopherol-coated
nanoparticles to encapsulate nerve growth factor
and evaluation of biodistribution", EUROPEAN
JOURNAL OF PHARMACEUTICS AND
BIOPHARMACEUTICS., vol. 108, 1 November
2016 (2016-11-01), NL, pages 126 - 135,
XP055548697, ISSN: 0939-6411, DOI:
10.1016/j.ejpb.2016.08.005

**Description**

La présente invention concerne un complexe sous la forme d'un antioxydant greffé sur un réservoir d'électrons autorégénéré en présence de photons. Il peut s'agir notamment de colloïdes d'oxyde de zinc ayant été greffés avec un antioxydant.

Le domaine d'utilisation de ces colloïdes concerne notamment la cosmétique, par exemple les compositions solaires, anti-âge et les compléments alimentaires. Il peut s'agir de la protection topique contre les effets néfastes du tabagisme, d'un régime riche en sucres ou induits par une exposition à un rayonnement ultraviolet et/ou à des molécules polluantes présentes dans l'atmosphère.

La peau est un organe en renouvellement permanent qui est soumis aux effets du temps. Au fur et à mesure que la peau vieillit, le renouvellement des cellules cutanées ralentit. Les cellules les plus anciennes s'accumulent et donnent l'impression de teint brouillé, la peau se dessèche et s'amincit. Parallèlement, apparaissent des modifications du derme, une fonte des tissus graisseux et musculaires et donc une perte de soutien. Il s'agit alors du processus de vieillissement cutané.

D'un point de vue moléculaire, le vieillissement cutané est lié à une altération des mécanismes de réparation des cellules. Il est déterminé, d'une part, par une « horloge biologique » individuelle génétiquement programmée ; et d'autre part, par les capacités de résistance de la cellule aux dégâts oxydatifs causés par des substances toxiques appelées radicaux libres. Il s'agit du vieillissement d'origine intrinsèque.

En parallèle, un vieillissement d'origine extrinsèque est induit par les facteurs environnementaux et les comportements propres à chaque individu. A titre d'exemple, ces facteurs extrinsèques regroupent le tabagisme, les polluants atmosphériques naturels ou artificiels, le stress, l'exposition au soleil, la consommation d'alcool ou encore un régime riche en sucres.

Par polluants atmosphériques naturels, on désigne les molécules et particules polluantes générées par les activités naturelles (volcans, minerais, océans,...), telles que le dioxyde de soufre ou encore l'ozone.

Par polluants atmosphériques artificiels, on désigne les molécules et particules polluantes générées par les activités anthropiques (usines, moteurs de voitures,...), telles que les hydrocarbures aromatiques polycycliques, les métaux lourds ou encore les pesticides.

Certaines de ces molécules polluantes peuvent être à l'origine d'un phénomène d'inflammation, de vieillissement cellulaire accéléré, voire même de certains cancers cutanés. Parmi ces composés, les polluants organiques persistants font partie des polluants atmosphériques les plus dangereux pour la santé. Ils se fixent généralement à la surface de la peau. A la suite de leur absorption, un phénomène de stress oxydatif se met en place et est à l'origine de l'accélération du processus de vieillissement cutané.

De la même manière, le rayonnement UV conduit notamment, de façon directe ou indirecte, à l'apparition d'un déséquilibre entre les éléments prooxydants et les antioxydants conduisant à l'apparition d'un phénomène de stress oxydatif au sein des cellules, par exemple des cellules de la peau.

Un régime alimentaire riche en sucres peut conduire à l'apparition de protéines glyquées qui ne peuvent être ni détruites, ni libérées de la cellule. Il s'ensuit une production accrue de radicaux libres qui crée un déséquilibre en faveur de l'activité prooxydante et donc du vieillissement physiologique de l'organisme.

La consommation d'alcool et/ou de tabac modifie le métabolisme des cellules et augmente la production de radicaux libres ce qui crée un déséquilibre entre la quantité de ces radicaux libres produits et le système de défense antioxydant.

Un radical libre peut être défini comme une espèce chimique (molécule ou atome) possédant un ou plusieurs électrons non appariés sur sa couche externe. Les plus fréquents sont les radicaux hydroxyles ($OH^•$), superoxydes ($O_2^{•-}$), oxydes nitriques ($NO^*$), thiols ($RS^*$) ou encore peroxyles ($RO_2^{•}$). Si l'atome du radical libre correspond à un oxygène, les radicaux seront appelés « dérivés réactifs de l'oxygène » (*reactive oxygen species* ou ROS), tels que l'oxygène singulet ($^1O_2$) ou encore l'anion superoxyde ($O_2^{•-}$).

Les radicaux libres sont des molécules chimiques instables en raison du ou des électrons non appariés qui leurs procurent une grande réactivité vis-à-vis des molécules environnantes. Un radical libre est un élément prooxydant qui est neutralisé au détriment de la molécule voisine qui devient à son tour un radical libre et ainsi de suite. Le phénomène se propage par des réactions en chaîne, il s'agit du stress oxydatif.

En pratique, chaque molécule prooxydante nécessite l'apport d'au moins un voire de deux ou trois électrons pour se stabiliser, c'est-à-dire être neutralisé.

Quotidiennement, la peau est confrontée à des agressions environnementales. Pour se défendre de ces attaques, le corps humain a développé des systèmes de défense via l'utilisation d'agents antioxydants enzymatiques et/ou non-enzymatiques. Cependant, quand une quantité excessive de radicaux libres est générée, un déséquilibre s'installe et le système de défense antioxydant se trouve submergé conduisant à créer des dommages du tissu cutané.

Les antioxydants peuvent être définis comme toute substance qui, présente à faible concentration par rapport au substrat oxydable, est capable de ralentir, au moins, l'oxydation de ce substrat.

Cette définition fonctionnelle s'applique à un grand nombre de substances, comprenant des enzymes aux propriétés catalytiques spécifiques, mais aussi de petites molécules hydrophiles ou liposolubles. Cette grande variété physico-chimique autorise la présence d'antioxydants dans tous les compartiments de l'organisme, qu'ils soient intracellulaires, membranaires ou extracellulaires.

A titre d'exemple, parmi les antioxydants enzymatiques sont listés la superoxyde dismutase ou la glutathion peroxydase et parmi les antioxydants non-enzymatiques, on retrouve des vitamines (A, C, E), des oligoéléments (le sélénium) ou encore des protéines (la ferritine).

L'activité des agents antioxydants permet de ralentir, retarder ou prévenir la réaction en chaîne prooxydante initiée par les radicaux libres. Il s'agit donc d'agents de prévention ou de terminaison capables d'éviter ou de piéger les radicaux libres. En d'autres termes, les antioxydants transforment les radicaux en composés plus stables et bloquent la phase de propagation *via* le transfert de multi-électrons aux éléments prooxydants pour les stabiliser partiellement ou totalement.

Le complexe de l'invention présente une activité antioxydante mimant celle de la superoxyde dismutase (SOD).

L'activité de la SOD correspond à une activité catalytique de la dismutation de l'anion superoxyde ($O_2^{\bullet-}$), en dioxygène ($O_2$) et peroxyde d'hydrogène ($H_2O_2$). Les produits de cette réaction sont considérés comme des radicaux oxygénés libres, même s'ils ne possèdent pas d'électrons non appariés, et s'avèrent être très réactifs et nocifs. A titre d'exemple, en présence de fer, le peroxyde d'hydrogène va se décomposer et produire un radical hydroxyle OH* très toxique envers la plupart des structures organiques. Ces éléments sont alors considérés comme des ROS secondaires produits par la réaction de la dismutation de l'anion superoxyde.

En conditions physiologiques, cette dismutation est lente et conduit à une demi-vie prolongée de l'anion superoxyde produisant une oxydation délétère des macromolécules biologiques par la production desdits ROS secondaires. Or, la toxicité de ces ROS est largement supérieure à celle de l'anion superoxyde.

La SOD va catalyser et donc accélérer la dismutation de l'anion superoxyde pour réduire la demi-vie de cet élément et donc limiter les effets prooxydants des ROS produits secondairement à la réaction de la dismutation de l'anion superoxyde.

En outre, la SOD va également permettre l'élimination d'autres ROS, à titre d'exemple, $^1O_2$ ou encore ROO.

En effet, la SOD est une enzyme capable de réduire un agent prooxydant par transfert de multi-électrons. En d'autres termes, la SOD peut réagir avec tous les ROS pour les stabiliser et prévenir ou neutraliser leur activité prooxydante.

Cette protéine fournit donc une activité catalytique essentielle au mécanisme d'élimination des ROS et donc du stress oxydatif.

En cosmétique, de nombreux produits visent à produire un effet antioxydant, aussi dénommé effet anti-radicalaire, pour lutter contre le vieillissement d'origine intrinsèque et/ou extrinsèque

Par exemple le document US2003/049287A1 décrit le dérivé catéchol oxime comme agent anti-radicalaire et antioxydant.

La protection contre le stress oxydatif, notamment induit par les facteurs extrinsèques, est actuellement basée sur l'exploitation des vertus antioxydantes par exemple d'extraits végétaux. Cependant, ces formulations sont complexes et mobilisent un nombre très important d'ingrédients, entre autres des solvants et conservateurs, dont l'innocuité n'est pas toujours avérée à court ou à long terme.

Ainsi et en dépit de leur formulation, il apparait que l'efficacité de la grande majorité des solutions proposées n'est pas suffisante.

Les dérivés de molécules antioxydantes sont plus couramment utilisés en formulation pharmaceutique. Cependant, ils présentent l'inconvénient d'être moins efficaces et moins disponibles par rapport aux antioxydants à l'état natif et à dose équivalente.

Les solutions conventionnelles pour lutter contre les radicaux libres et leurs conséquences nécessitent une application fréquemment renouvelée pour assurer un effet protecteur et/ou réparateur régulier et continu dans le temps.

A l'heure actuelle, aucune composition cosmétique ne propose une formulation comprenant une molécule enzymatique dont l'activité est stable dans le temps, pour oxyder tous les types de ROS et diminuer ou annihiler le stress oxydatif.

Pour résoudre ce problème, le Demandeur a mis au point un complexe mimant l'effet de la SOD.

Ce complexe résulte de la combinaison d'un antioxydant et de colloïdes, servant de réservoir d'électrons, permettant ainsi de lutter contre les effets néfastes de tous les radicaux libres.

Plus précisément, la présente invention concerne des colloïdes semi-conducteurs greffés de manière covalente, c'est-à-dire par l'intermédiaire d'au moins une liaison covalente ou bras espaceur, avec un antioxydant, à savoir un aldéhyde phénolique ou un acide phénolique comme définit dans les revendications.

Ces colloïdes semi-conducteurs sont constitués d'au moins un élément choisi dans le groupe comprenant Cd, Zn, Cu, Ti, Mn, Fe, Ni, Cr Hg, et leurs assemblages.

De manière avantageuse, les colloïdes semi-conducteurs selon l'invention comprennent deux, ou trois de ces éléments.

Selon un mode de réalisation particulier, les colloïdes semi-conducteurs selon l'invention sont des colloïdes d'oxyde de zinc, ZnO.

Selon un mode de réalisation particulier, les colloïdes semi-conducteurs selon l'invention sont des colloïdes de dioxyde de titane rutile, $TiO_2$.

De manière générale par « colloïde semi-conducteur », on désigne un composé ayant une différence d'énergie entre la bande de valence et la bande de conduction suffisamment faible pour qu'un électron passe de l'une à l'autre.

Au sens de l'invention, par « antioxydant » on désigne une molécule organique capable de donner au moins 1 électron ou H* à une espèce chimique. Selon l'invention, cet antioxydant porte au moins une fonction capable de réagir avec une fonction disponible présente à la surface des colloïdes semi-conducteurs pour créer le complexe selon l'invention.

Le greffage de l'antioxydant s'effectue par formation de liaison(s) chimique(s) covalente(s) désignées ensuite bras espaceur ou précurseur entre les colloïdes et l'antioxydant.

Le complexe de l'invention comprend 2 éléments, respectivement, un colloïde et un antioxydant reliés par une liaison covalente. Le colloïde agit en tant que réservoir d'électrons, c'est-à-dire qu'il est capable de transférer à l'antioxydant, du fait de sa proximité, un électron et ce, tant que le réservoir en contient et tant que l'antioxydant est demandeur. L'électron ainsi transféré permet de régénérer la fonction anti-radicalaire de l'élément antioxydant. Le complexe de l'invention présente ainsi une activité mimant celle de la SOD, c'est-à-dire qu'il est capable de réduire un agent prooxydant au moyen de plusieurs électrons.

Selon une autre caractéristique, dans le cas d'une application topique, moyennant exposition à un rayonnement électromagnétique, par exemple des rayons ultraviolets (UV), notamment entre 280 et 400 nm dans le domaine des UV-A et des UV-B, il y aura régénération électronique du colloïde par conversion de l'énergie photonique en électrons. Cela signifie que le réservoir aura la capacité de donner indéfiniment des électrons à l'élément antioxydant.

En synthèse, les colloïdes constituent un réservoir d'électrons permettant de régénérer l'activité anti-radicalaire de l'antioxydant qui peut ainsi réagir avec autant de radicaux libres qu'il y a d'électrons disponibles dans les colloïdes et/ou pour oxyder et donc neutraliser tous les composés radicalaires. Cet effet est lié à la proximité entre les colloïdes et l'antioxydant. En effet, en l'absence de greffage, l'antioxydant, AntiOx, n'est pas régénéré même lorsque des colloïdes sont présents dans le milieu réactionnel.

Les colloïdes, également désignés « Col », sont des particules cristallines (forme non amorphe) ayant des propriétés semi-conductrices résultant de l'empilement ordonné de molécules, par exemples de molécules de ZnO. Ces colloïdes peuvent également être appelés « quantum dots » ou nanocristaux. Ils peuvent se présenter sous la forme d'une dispersion ou d'une suspension, avantageusement d'une dispersion, de colloïdes dans un milieu aqueux.

Les colloïdes peuvent être synthétisés selon les techniques conventionnelles, par exemple par l'approche dite « bottom-up » de croissance de précurseurs. Cette voie de synthèse, couramment utilisée dans le domaine des nanomatériaux, met en oeuvre une étape de nucléation et une étape de croissance à partir d'atomes isolés. Elle permet de contrôler la taille des colloïdes à l'échelle nanométrique.

Pour plus de clarté, le terme « Col/AntiOx » est utilisé pour désigner les colloïdes selon l'invention, c'est-à-dire des colloïdes, par exemples d'oxyde de zinc, greffés avec un antioxydant. En outre, « AntiOx » désigne l'antioxydant greffé sur les colloïdes.

La notion de greffage, ou de fonctionnalisation, des colloïdes fait partie des connaissances générales de l'homme du métier. Le greffage, ou fonctionnalisation, correspond à la formation de liaisons covalentes entre l'antioxydant et la surface des colloïdes. Le greffage de l'antioxydant sur le colloïde via un bras espaceur positionné entre le colloïde semi-conducteur et l'antioxydant, confère la proximité nécessaire au transfert multi-électron des colloïdes vers l'antioxydant.

Les colloïdes Col/AntiOx présentent l'avantage de conférer un effet antioxydant auto-régénérant en présence de molécules organiques, par exemple des radicaux organiques ou radicaux libres.

Avantageusement, l'antioxydant AntiOx greffé est plus stable. En d'autres termes, la demi-vie de l'antioxydant AntiOx est augmentée.

La présence des colloïdes Col/AntiOx permet d'éviter la formation d'espèces radicalaires secondaires à la réaction, notamment l'oxygène singulet et/ ou le peroxyde d'hydrogène. Au contraire, les compositions de l'art antérieur ne bloquent pas la formation de composés radicalaires secondaires, ce qui se traduit par la formation d'un déséquilibre entre agents prooxydants et antioxydants, produisant un stress oxydatif « secondaire » et des dommages cellulaires.

En d'autres termes, les colloïdes Col/AntiOx selon l'invention neutralisent toute activité de prooxydation primaire et/ou secondaire.

De manière générale, les colloïdes présentent une taille moyenne de l'ordre de quelques nanomètres à quelques dizaines de nanomètres.

Ainsi, les colloïdes présentent une taille avantageusement comprise entre 0,5 nm et 1000 nm, plus avantageusement entre 10 nm et 100 nm, et encore plus avantageusement de l'ordre de 30 nm, la taille étant mesurée par DRX.

La technique DRX (diffraction des rayons X) est une technique conventionnellement utilisée pour mesurer la taille de cristaux à l'état solide.

Par taille, on entend la dimension la plus importante des colloïdes (Col ou Col/AntiOx), par exemple le diamètre dans le cas de colloïdes de forme sphérique. Il s'agit de la taille moyenne de colloïdes greffés (Col/AntiOx) ou non (Col). En effet, la taille des colloïdes greffés Col/AntiOx est généralement comprise dans les plages de valeurs données ci-dessus. Le cas échéant, l'homme du métier saura adapter la taille des colloïdes non greffés Col.

Les colloïdes Col et/ou Col/AntiOx sont avantageusement de forme sphérique.

Bien entendu, le greffage ne se limite pas au greffage d'une seule molécule d'un seul antioxydant sur un colloïde. Il s'agit du greffage d'une multitude de molécules d'au moins un type d'antioxydant AntiOx sur chaque nanocristal, par exemple d'oxyde de zinc.

L'antioxydant AntiOx est une molécule capable de donner au moins un électron. l'antioxydant est un aldéhyde phénolique ou un acide phénolique.

A titre d'exemple, l'antioxydant AntiOx est dans ce cas 2-hydroxybenzaldéhyde, 3-hydroxybenzaldéhyde, le 4-hydroxybenzaldéhyde, le 3,4 dihydroxybenzaldéhyde, le 2,3 dihydroxybenzaldéhyde, le 4,5 dihydroxybenzaldéhyde, avantageusement le 3-hydroxybenzaldéhyde ou le 3,4 dihydroxybenzaldéhyde (aldéhyde protocatéchique).

Avantageusement et toujours selon ce mode de réalisation, le bras espaceur comprend de 1 à 8 carbones, de préférence 2 à 4 carbones et présente une fonction alkoxysilane capable de se lier de façon covalente au colloïde et une fonction de type hydroxyle, phosphate ou amine capable de se lier à l'antioxydant.

Avantageusement, le bras espaceur est un dérivé de silice, avantageusement un alkoxysilane, encore plus avantageusement il s'agit du 3-(Aminopropyl)triethoxysilane. Avantageusement, le ratio colloïde/dérivé de silice utilisé pour former le bras espaceur est comprise entre 1/1 et 10/1, de préférence 2/1 et 3/1.

Bien entendu, le bras espaceur sera choisi en fonction de la nature de l'antioxydant de sorte à assurer la liaison covalente et à ce que la proximité entre Col et AntiOx soit suffisante pour permettre le transfert multi-électron des colloïdes vers l'antioxydant.

Avantageusement, les colloïdes greffés avec un antioxydant selon l'invention sont des colloïdes de ZnO ou de TiO2 greffés avec du 3-hydroxybenzaldéhyde.

Le greffage covalent de l'antioxydant AntiOx est réalisé de manière conventionnelle par réaction entre une fonction de l'antioxydant AntiOx ou un précurseur de l'antioxydant AntiOx et des fonctions disponibles en surface des colloïdes Col.

Les colloïdes ne sont pas dopés. Eventuellement, ils peuvent comprendre un métal de transition qui est introduit lors de la synthèse des colloïdes.

Par « dopage », on désigne l'incorporation d'un élément au sein d'un matériau déjà formé. L'incorporation d'un élément lors de la synthèse d'un matériau, c'est à dire en amont de la formation du matériau, n'est pas considérée comme étant du dopage.

La présente invention concerne également l'utilisation des colloïdes greffés avec au moins un antioxydant.

Les colloïdes Col/AntiOx peuvent être utilisés dans une composition topique, par exemple une composition cosmétique mais aussi, dans une composition cosmétique à usage non thérapeutique. Cette composition est avantageusement hydrophile, micellaire ou Pickering (forme inversée d'une composition micellaire).

Lorsque la composition est à usage topique, la formulation est avantageusement dépourvue de filtres solaires lipophiles ou hydrophiles, minéraux ou organiques. En effet, leur présence conduirait à bloquer le passage des UV nécessaires et indispensables à l'obtention des effets antioxydant et d'autorégénération tels que décrits.

La composition cosmétique selon l'invention peut également comprendre les adjuvants habituels dans le domaine considéré.

Bien entendu, l'homme du métier veillera à choisir ce, ou ces éventuels adjuvants ou excipients complémentaires, et/ou leur quantité, de manière telle que les propriétés avantageuses de la composition selon l'invention ne soient pas, ou substantiellement pas, altérées par l'adjonction envisagée.

Les colloïdes Col/AntiOx peuvent également être utilisés dans un complément alimentaire, par exemple un probiotique.

Lorsque les complexes sont utilisés en tant que complément alimentaire, l'élément antioxydant n'est régénéré qu'à hauteur du contenu en électrons du réservoir, c'est-à-dire du colloïde. En effet, dans ce cas, les colloïdes ne sont pas exposés à un rayonnement ultraviolet, ce qui empêche leur régénération. En revanche, l'antioxydant AntiOx bénéficie du réservoir d'électrons constitué par les colloïdes, augmentant ainsi sa demi-vie et son efficacité.

Le complément alimentaire comprenant Col/AntiOx peut comprendre des excipients conventionnels. Il peut notamment se présenter sous la forme de gélules ou de comprimés. L'invention et les avantages qui en découlent ressortiront mieux des figures et exemples suivants donnés afin d'illustrer l'invention et non de manière limitative.

EP 3 801 058 B1

**DESCRIPTION DES FIGURES**

La figure 1 représente le diffractogramme de colloïdes de ZnO avant fonctionnalisation avec l'antioxydant. Les raies sont indexées avec les plans correspondant à la structure ZnO hexagonale.

La figure 2 représente la cinétique DPPH pour des colloïdes fonctionnalisés avec l'antioxydant (Col/AntiOx) et pour l'antioxydant seul.

La figure 3 représente le pourcentage de substrat ABTS dégradé par les colloïdes selon l'invention exposés aux rayons UV ou maintenus dans l'obscurité.

La figure 4 illustre l'activité antioxydante des colloïdes fonctionnalisés avec l'antioxydant (Col/AntiOx) selon l'invention par rapport au tocophérol, placés à l'obscurité ou exposé aux UV.

La figure 5 illustre l'activité antioxydante des colloïdes fonctionnalisés avec un antioxydant (Col/AntiOx) selon l'invention par rapport à d'autres antioxydants.

La figure 6 illustre l'inhibition de toute activité de prooxydation primaire et/ou secondaire des colloïdes fonctionnalisés avec l'antioxydant (Col/AntiOx) selon l'invention par rapport à d'autres antioxydants.

**EXEMPLES DE REALISATION DE L'INVENTION**

*Exemple 1 : ZnO/3-hydroxybenzaldéhyde*

1/ Synthèse des colloïdes

Les pourcentages sont donnés en poids de la composition.

Un précurseur d'oxyde de zinc, tel que l'acétate de zinc anhydre (2%) et de l'hydroxyde de sodium (1.5%), est mélangé avec un solvant ou un mélange de solvants éthanol (85%)/diethylene glycol (8%) et scellés dans un autoclave. Le solvant peut également être de alcool benzylique, phénol, alcool oléylique, butanol, propanol, isopropanol, eau, tétrahydrofurane, éthanol, méthanol, acétonitrile, toluène, PGMEA, PGPE, PGME, 2-méthyl-1-propanol ou éther monométhylique de triéthylèneglycol.

Le milieu réactionnel est placé sous agitation mécanique à 60°C pendant 30 minutes environ, jusqu'à dissolution des sels. On ajoute ensuite de l'eau (2%). On observe l'apparition d'un trouble qui marque le début de la formation des particules. La réaction est maintenue à 65°C pendant 1H30.

Des nanocristaux d'oxyde de zinc sphériques de 7 nm de diamètre sont récoltés.

2/ Préparation des colloïdes Col/AntiOx

Des colloïdes selon l'invention ont été préparés à partir des colloïdes d'oxyde de zinc (ZnO) obtenus ci-avant et d'un précurseur ou bras espaceur de l'antioxydant 3-hydroxybenzaldéhyde. On obtient ainsi, des particules de ZnO/3-hydroxybenzaldéhyde. En pratique, les nano cristaux formés sont fonctionnalisés in-situ avec du (3-Aminopropyl)triethoxysilane (0.5%) A l'ajout, la solution s'éclaircit légèrement. Cette fonctionnalisation se fait pendant 3H00 supplémentaires. On ajoute enfin l'antioxydant 3-Hydroxybenzaldehyde (1.5%). La réaction est maintenue pendant 9 heures.

Le réacteur est ensuite refroidi à température ambiante. Les particules sont récupérées et centrifugées à 3000 tours/minute pendant 15 minutes. Elles sont ensuite lavées dans de l'éthanol, puis à nouveau centrifugées. Enfin, les particules sont dispersées à la concentration voulue dans l'eau (concentration entre 10 et 15% massique).

La faible taille des particules de ZnO permet de disposer d'une aire plus importante à couvrir et donc de greffer un plus grand nombre de molécules antioxydantes à la surface de chacune des particules.

Le diamètre des colloïdes ZnO a été mesuré au moyen d'un diffractomètre à rayon X (DRX). La longueur d'onde produite par le diffractomètre correspond à la raie Cu-K$\alpha$ égale à 1,54 Å. Les autres paramètres utilisés correspondent à une tension d'accélération de 40kV, un courant électrique de 40 mA et une géométrie de Bragg-Brentano.

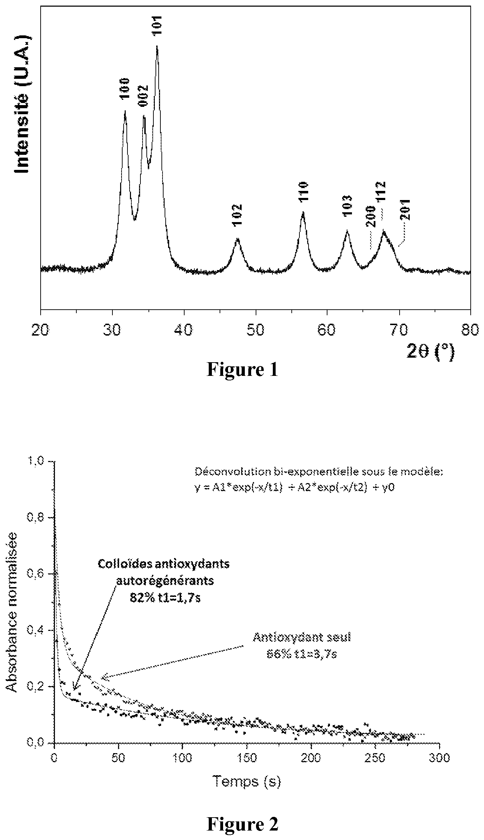

Les diffractogrammes ont été mesurés sur poudre avec un DRX de source Cu-K$\alpha$ en transmission. Le diffractogramme de colloïdes avant fonctionnalisation (greffage) avec l'antioxydant est représenté par la figure 1.

*Exemple 2 : TiO$_2$/3-hydroxybenzaldéhyde*

Dans les mêmes conditions que dans l'exemple précédent, on greffe le même antioxydant sur du dioxyde de titane rutile. On disperse 5g de dioxyde de titane rutile dans un mélange éthanol 950 mL et de Diéthylene glycol 50 mL. Le mélange est chauffé à 65°C et agité. 5 mL d'eau sont ajoutés puis 8 mL de (3-Aminopropyl)triethoxysilane. Le mélange

6

est laissé agité deux heures puis 5 g de 3-hydroxybenzaldéhyde sont ajoutés et le mélange est laissé agité et chauffé à 65°C pendant 9 heures. Le mélange est ensuite refroidi à température ambiante. Les particules sont récupérées et centrifugées à 3000 tours/minute pendant 15 minutes. Elles sont ensuite lavées dans de l'éthanol, puis à nouveau centrifugées. Enfin, les particules sont dispersées à la concentration voulue dans l'eau. Des synthèses similaires peuvent être conduites en mettant en oeuvre en tant qu'antioxydant 2-hydroxybenzaldéhyde ou le 4-hydroxybenzaldéhyde.

*Exemple 3 : TiO$_2$/3,4 dihydroxybenzaldéhyde*

Dans les mêmes conditions que dans l'exemple précédent, on greffe le 3,4 dihydroxybenzaldéhyde sur du dioxyde de titane rutile. On disperse 5g de dioxyde de titane rutile dans un mélange éthanol 950 mL et de Diéthylene glycol 50 mL. Le mélange est chauffé à 65°C et agité. 5 mL d'eau sont ajoutés puis 8 mL de (3-Aminopropyl)triethoxysilane. Le mélange est laissé agité deux heures puis 5 g de 3,4 dihydroxybenzaldéhyde sont ajoutés et le mélange est laissé agité et chauffé à 65°C pendant 9 heures. Le mélange est ensuite refroidi à température ambiante. Les particules sont récupérées et centrifugées à 3000 tours/minute pendant 15 minutes. Elles sont ensuite lavées dans de l'éthanol, puis à nouveau centrifugées. Enfin, les particules sont dispersées à la concentration voulue dans l'eau.

*Exemple 4 : Comparaison du temps de cinétique de l'activité antioxydante des colloïdes Col/AntiOx (ZnO/3-hydroxybenzaldéhyde) avec celui d'un agent antioxydant libre (3-hydroxybenzaldéhyde)*

La vitesse d'action des colloïdes Col/AntiOx a été estimée en mesurant la cinétique de décomposition du 2,2-diphényl-1-picrylhydrazyl (DPPH).
Le DPPH est une molécule qui conserve sa qualité de radical libre de manière stable. Cette espèce radicalaire absorbe la lumière à 520 nm (couleur violette de la solution) et devient incolore ou jaune pâle après neutralisation par un antioxydant. Il est ainsi possible de suivre la réaction de neutralisation en mesurant l'intensité de la mesure d'absorption du DPPH radicalaire en fonction du temps.
Pour cela, deux solutions dans l'éthanol sont préparées comme suit :

- une solution témoin contenant du DPPH de concentration [DPPH]$_0$ de 0,1 mol/L.
- une solution test contenant du DPPH de concentration [DPPH]$_0$ de 0,1 mol/L et un antioxydant à une concentration telle que 90% de DPPH soit consommé après 2h (détermination à partir des mesures CI$_{50}$).

L'absorbance est mesurée à l'aide d'un spectrophotomètre UV/VIS/NIR. Dans une cuve en polystyrène (trajet optique = 1 cm), sont versées 2,5 mL de solution. Le spectre d'absorption UV/visible entre 310 et 700 nm est mesuré pendant 300 secondes. La valeur de l'absorbance à 520 nm permet de déterminer la concentration de DPPH radicalaire à un temps donné selon l'équation de Beer-Lambert :

$$A_{520nm}(\tau) = \varepsilon_{DPPH}.l.[DPPH]_\tau$$

Selon cette équation, A correspond à l'absorbance mesurée, $\varepsilon_{DPPH}$ correspond au coefficient massique du DPPH, 1 (cm) correspond au trajet optique à travers l'échantillon et [DPPH]$_\tau$ (g/L) correspond à la concentration massique de l'échantillon.
Les courbes obtenues sont affinées selon un modèle cinétique qui permet de remonter à la constante cinétique $\kappa$ et la constante de demi-vie $\tau_{1/2}$ de la réaction.
Le graphique démontrant la vitesse d'action des colloïdes fonctionnalisés avec un antioxydant (ZnO/3-hydroxybenzaldéhyde) et celle de l'antioxydant libre (3-hydroxybenzaldéhyde), c'est-à-dire non greffé, est représenté par la figure 2.
Les résultats montrent que l'antioxydant de ZnO/3-hydroxybenzaldéhyde réagit plus rapidement avec le DPPH que le 3-hydroxybenzaldéhyde libre.

*Exemple 5 : Evaluation de la régénération de l'activité antioxydante des colloïdes Col/AntiOx*

*5.1/ Evaluation au cours du temps*

Une solution stock d'ABTS (acide 2,2'-azino-bis(3-éthylbenzothiazoline-6-sulphonique)) à 8mM est incubée, à volume égal, avec une solution à 3mM comprenant de la métmyoglobine et du peroxyde d'hydrogène pour produire un radical ABTS cationique. La solution obtenue est diluée avec du tampon phosphate (0,2 M, et pH 7,4) contenant 150 mM de NaCl pour obtenir une absorbance de 1,5 à 734 nm.

Des échantillons de 30 μL de ZnO/3-hydroxybenzaldéhyde (exemple 2/) dispersés à 240 g/L dans l'eau sont ajoutés à 2970 μL de la solution cationique d'ABTS 0,07 mM dans l'eau, puis placés sous agitation dans le noir.

Après 30 minutes d'incubation, l'ABTS est totalement dégradé.

La solution obtenue après cette étape de dégradation de tout le substrat ABTS est séparée : une partie de la solution est placée dans le noir, l'autre partie est placée sous irradiation UV. Après 30 minutes d'exposition (à l'obscurité ou aux UV), 30 μL d'une solution concentrée d'ABTS (7 mM) sont ajoutés, puis les solutions sont à nouveau placées à l'obscurité sous agitation pendant 5 heures. L'absorption est ensuite mesurée toutes les 30 minutes.

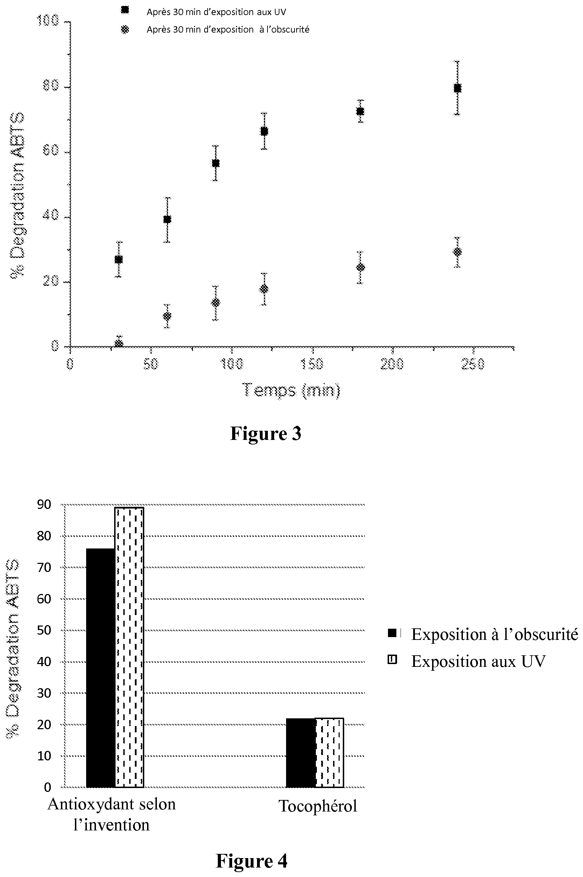

Les données sont représentées par la figure 3.

Les résultats montrent que la solution contenant des colloïdes ZnO/3-hydroxybenzaldéhyde et exposée aux rayons UV présente une capacité de dégradation de l'ABTS largement supérieure à celle contenant des colloïdes ZnO/3-hydroxybenzaldéhyde mais restée à l'obscurité. L'activité de dégradation de l'ABTS observée pour la solution placée à l'obscurité correspond à l'activité anti-radicalaire résiduelle inhérente au complexe ZnO/3-hydroxybenzaldéhyde. Ainsi, après 120 minutes, 65% d'ABTS sont dégradés par la solution ayant été exposée au rayonnement UV contre 18% pour la solution restée dans le noir.

En conclusion, les colloïdes ZnO/3-hydroxybenzaldéhyde selon l'invention possède la capacité de régénérer leur activité antioxydante lorsqu'ils sont exposés aux rayonnements UV.

*5.21 Comparaison avec le tocophérol*

Le protocole de préparation des solutions selon le point 4.1/ a été répété avec une concentration des colloïdes ZnO/3-hydroxybenzaldéhyde de 0,5 g/L et une concentration de tocophérol de 1,5 g/L.

Les solutions obtenues sont séparées : une partie de la solution est placée dans le noir, l'autre partie est placée sous irradiation UV. Après 30 minutes d'exposition (à l'obscurité ou aux UV), 30 μL d'une solution concentrée d'ABTS (7 mM) sont ajoutés, puis l'absorption est ensuite mesurée.

Les données sont représentées par la figure 4.

Les résultats montrent que la solution contenant du tocophérol présente la même capacité de dégradation de l'ABTS (22%) après une exposition aux UV ou à l'obscurité.

La solution contenant des colloïdes ZnO/3-hydroxybenzaldéhyde exposée à l'obscurité présente une capacité de dégradation de l'ABTS très largement supérieure au tocophérol (76%). Cette activité est encore augmentée après une exposition aux UV (89%).

En conclusion, les colloïdes ZnO/3-hydroxybenzaldéhyde selon l'invention possèdent une activité anti-radicalaire plus efficace qu'un antioxydant conventionnel et une capacité à régénérer l'activité antioxydante lorsqu'ils sont exposés à un rayonnement UV.

*Exemple 6 : Evaluation de l'activité antioxydante des colloïdes fonctionnalisés avec antioxydants (ZnO/3-hydroxybenzaldéhyde) selon l'invention*

L'activité antioxydante des colloïdes ZnO/3-hydroxybenzaldéhyde a été estimée en mesurant la décomposition du 2,2-diphényl-1-picrylhydrazyl (DPPH).

L'expression des résultats se fait en $CI_{50}$ et/ou en mg ou μmol équivalent de l'échantillon antioxydant sélectionné. Cette méthode témoigne de l'aptitude d'une molécule à piéger les radicaux libres par transfert d'électrons et/ou de protons issus de phénomènes d'oxydations. Le protocole est le suivant :

- une solution DPPH éthanolique est préparée à 0,1 mM,
- les échantillons (antioxydant) à tester sont préparés à différentes masses comprises entre 4 mg et 40 mg,
- dans une cuve de PMMA (poly(méthacrylate de méthyle)), 3900 μL de solution de DPPH sont mélangés à 100 μL de l'échantillon à tester. La réaction est réalisée sous incubation à température ambiante, pendant 2 heures, puis l'absorbance à 515 nm est mesurée,
- le pourcentage de l'activité antioxydante est tracé en fonction de la concentration de l'échantillon considéré (en μM).

Le pourcentage de l'activité antioxydante est déterminé selon la formule suivante :

$$\%activité\ antioxydante = 1 - (A_{éch}\ /\ A_{blanc}) \times 100$$

$A_{éch}$ correspond à l'absorbance de l'échantillon alors que $A_{blanc}$ correspond à l'absorbance d'une solution sans échantillon (100 μL éthanol + 3900 μL d'une solution DPPH/éthanol).

La courbe obtenue présente une partie linéaire et une partie asymptotique. La zone linéaire est de la forme %

AO = $\chi \times$ [échantillon].

Cette formule permet de déterminer la concentration en échantillon qui permet de piéger 50% des radicaux présents, soit de déterminer la CI$_{50}$ en g/L (concentration minimale inhibitrice à 50%) de l'échantillon considéré :

$$CI_{50} = 50 / \chi$$

$\chi$ représentant la pente de la régression linéaire.

L'activité antioxydante des colloïdes selon l'exemple 2 (ZnO/3-hydroxybenzaldéhyde) a été comparée à celle d'agents antioxydants conventionnels listés ci-après :

- Echantillon de 30 μL dilués à 0,5 g/L sont placés dans 2970 L d'une solution à 0,07 mM d'ABTS,
- Extrait de thé vert contenant plus de 40% de gallate d'épigallocatéchine (EGCG),
- 87,3% d'α-tocophérol + 12,7% huile de soja,
- 100% de L-acide ascorbique (Vitamine C),
- 100% de gallate de propyle

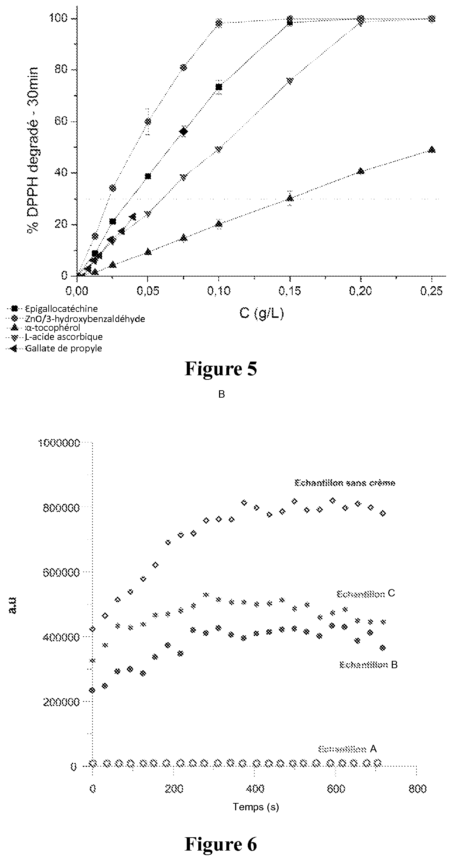

Les résultats sont représentés par la figure 5.

La figure 5 montre que les colloïdes selon l'invention présentent une CI$_{50}$ d'environ 0,03 g/L, soit très inférieure à celle des agents antioxydants conventionnels. En d'autres termes, la concentration nécessaire de ZnO/3-hydroxybenzaldéhyde selon l'invention pour piéger 50% des radicaux libres est inférieure aux antioxydants conventionnels.

En outre, une activité antioxydante supérieure à celle des autres antioxydants à concentration inférieure ou égale à 0,15 g/L et similaire à concentration supérieure à 0,15 g/L est observée.

L'activité antioxydante de ZnO/3-hydroxybenzaldéhyde est donc supérieure à celle des autres agents antioxydants testés.

*Exemple 7 : Evaluation du pouvoir protecteur de la composition selon l'invention contre la génération de radicaux libres primaires et secondaires*

L'étude est réalisée par la méthode de « spin trapping » couplée à la spectroscopie par Résonance Paramagnétique Electronique (RPE) pour effectuer des expériences de piégeage en compétition en utilisant la DIPPMPO (5-(Diisopropoxyphosphoryl)-5-methyl-1-pyrroline-N-oxide) comme molécule piège. Ce composé est utilisé de manière conventionnelle pour détecter et étudier le radical superoxyde par cette technique.

L'évolution de l'intensité du signal de l'adduit DIPPMPO-OOH a été suivie en fonction du temps en présence ou non des crèmes. La méthode a été évaluée en étudiant la cinétique de décomposition d'un radical modèle (TEMPOL ou 1-Oxyl-2,2,6,6-tetramethyl-4-hydroxypiperidine) en présence ou non des crèmes. Sur la durée de l'étude, soit 20 minutes, aucune perte de signal du TEMPOL n'a été observée pour les 4 expériences (contrôle + 3 échantillons). Egalement, des analyses contrôle en présence de Superoxyde Dismutase (SOD) ont été effectuées.

Tableau 2 : Pourcentage d'inhibition du signal DIPPMPO-OOH en fonction du temps.

| Crème | Inhibition à 3 minutes | Inhibition à 5 minutes | Inhibition à 10 minutes | Inhibition à 15 minutes |
|---|---|---|---|---|
| **Echantillon A** Complexe selon l'invention (ZnO/3-hydroxybenzaldéhyde) | 100 % | 100 % | 100 % | 100 % |
| **Echantillon B** Mélange d'antioxydants : squalane + tocopherol acétate + magnésium ascorbyl phosphate | 48% | 50% | 45 % | 53 % |
| **Echantillon C** Mélange d'antioxydants : tocopherol acetate + tocopherol + ergothionéine | 20% | 23% | 37% | 44% |

Les valeurs reportées dans le tableau sont la moyenne de 3 expériences identiques (les fluctuations étant inférieures à 10%)

Les cinétiques d'inhibition sont représentées par la figure 6.

Des propriétés de piégeage du radical superoxyde différentes sont observées pour chaque échantillon.

L'échantillon B montre une diminution de 48% à 3 minutes (53% à 5 minutes) du signal de l'adduit DIPPMPO-OOH, laissant supposer une inhibition de moitié de l'activité oxydante.

L'échantillon C montre une diminution de 20% à 3 minutes (44% à 5 minutes) du signal de l'adduit DIPPMPO-OOH, laissant supposer une inhibition moins efficace que l'échantillon B.

Pour le complexe selon l'invention, soit l'échantillon A (les colloïdes ZnO/3-hydroxybenzaldéhyde), aucun signal correspondant à l'adduit DIPPMPO-OOH n'est observé. Ainsi, tout le radical superoxyde produit a été piégé.

Au cours de ces expériences, la formation d'un radical supplémentaire a été observée (excepté pour l'échantillon selon l'invention). Selon le Demandeur, il semble que ce nouveau radical provienne d'une réaction d'Haber Weiss ou de Fenton impliquant la présence de sels de métaux de transition disponibles pour réagir avec le radical superoxyde ou le peroxyde d'hydrogène (généré par la dismutation spontanée du radical superoxyde). Ainsi, le radical hydroxylé serait généré et réagirait avec les constituants de la crème pour produire un nouveau radical, dit radical secondaire, observé sous forme d'adduit sur la DIPPMPO.

En conclusion, les colloïdes selon l'invention présentent une capacité supérieure, aux autres composés testés, à piéger les radicaux libres de type superoxyde. Egalement, aucune production d'espèces radicalaires secondaires n'est observée avec les colloïdes selon l'invention démontrant une efficacité totale sur l'inhibition des ROS et donc du stress oxydatif.

## Revendications

1. Colloïdes semi-conducteurs greffés de manière covalente avec un antioxydant, **caractérisé en ce que** les colloïdes sont des colloïdes semi-conducteurs constitués d'au moins un élément choisi dans le groupe comprenant Cd, Zn, Cu, Ti, Mn, Fe, Ni, Cr, Hg, et leurs assemblages ; et **en ce que** l'antioxydant est un aldéhyde phénolique ou un acide phénolique.

2. Colloïdes selon la revendication 1, **caractérisés en ce que** les colloïdes semi-conducteurs sont des colloïdes d'oxyde de zinc, ZnO ou des colloïdes de dioxyde de titane rutile, $TiO_2$.

3. Colloïdes, selon l'une des revendications précédentes, **caractérisés en ce que** l'antioxydant est choisi dans le groupe comprenant le 2-hydroxybenzaldéhyde, 3-hydroxybenzaldéhyde, le 4-hydroxybenzaldéhyde, le 3,4 dihydroxybenzaldéhyde.

4. Colloïdes, selon la revendication 3, **caractérisés en ce que** l'antioxydant est le 3-hydroxybenzaldéhyde.

5. Colloïdes, selon la revendication 3, **caractérisés en ce que** l'antioxydant est 3,4 dihydroxybenzaldéhyde.

6. Colloïdes, selon l'une quelconque des revendications précédentes, **caractérisés en ce que** le greffage de manière covalente se présente sous la forme d'un bras espaceur positionné entre le colloïde semi-conducteur et l'antioxydant, le bras espaceur comprenant de 1 à 8 carbones, de préférence 2 à 4 carbones et présentant une fonction alkoxysilane capable de se lier de façon covalente au colloïde et une fonction de type hydroxyle, phosphate ou amine capable de se lier à l'antioxydant.

7. Colloïdes, selon la revendication précédente, **caractérisé en ce que** le bras espaceur est le 3-(Aminopropyl)triethoxysilane.

8. Composition topique comprenant des colloïdes selon l'une des revendications 1 à 7.

9. Composition topique à usage non thérapeutique comprenant des colloïdes selon l'une des revendications 1 à 7.

10. Composition topique, selon la revendication 8 ou 9, **caractérisée en ce qu'**elle est dépourvue de filtres solaires hydrophiles et/ou lipophiles, minéraux et/ou organiques.

11. Composition topique selon l'un des revendications 8 à 10, **caractérisée en ce qu'**elle est sous forme aqueuse, micellaire ou Pickering.

12. Complément alimentaire comprenant des colloïdes selon l'une des revendications 1 à 7.

**Patentansprüche**

1. Halbleiterkolloide, kovalent gebunden an ein Antioxydans, **dadurch gekennzeichnet, dass** es sich bei den Kolloiden um Halbleiterkolloide handelt, bestehend aus mindestens einem Element ausgewählt aus der Gruppe, die Cd, Zn, Cu, Ti, Mn, Fe, Ni, Cr, Hg und ihre Verbindungen umfasst; und dadurch, dass das Antioxydans ein Phenolaldehyd oder eine Phenolsäure ist.

2. Kolloide nach Anspruch 1, **dadurch gekennzeichnet, dass** es sich bei den Halbleiterkolloiden um Zinkoxidkolloide, ZnO oder des Rutil- Titandioxid- Kolloid, $TiO_2$, handelt.

3. Kolloide, nach einem der vorangehenden Ansprüche, **dadurch gekennzeichnet, dass** das Antioxydans ausgewählt wird aus der Gruppe zu der 2-hydroxybenzaldehyd, 3- hydroxybenzaldehyd, 4-hydroxybenzaldehyd und 3,4 dihydroxybenzaldehyd gehören.

4. Kolloide, nach Anspruch 3, **dadurch gekennzeichnet, dass** es sich bei dem Antioxydans um 3- hydroxybenzaldehyd handelt.

5. Kolloide, nach Anspruch 3, **dadurch gekennzeichnet, dass** es sich bei dem Antioxydans um 3,4 dihydroxybenzaldehyd handelt.

6. Kolloide, nach irgendeinem der vorstehenden Ansprüche, **dadurch gekennzeichnet, dass** die kovalente Verbindung die Form eines Spacers hat, angeordnet zwischen dem Halbleiterkolloid und dem Antioxydans, der Spacer umfasst dabei 1 bis 8 Kohlenstoffatome, vorzugsweise 2 bis 4 Kohlenstoffatome, mit einer Alkoxysilan- Funktion, die in der Lage ist, sich kovalent an das Kolloid zu binden sowie eine Funktion vom Typ Hydroxyl, Phosphat oder Amino, die sich an das Antioxydans binden kann.

7. Kolloide, nach dem vorangehenden Anspruch, **dadurch gekennzeichnet, dass** der Spacer 3-(Aminopropyl)triethoxysilan ist.

8. Topische Zusammensetzung, umfassend die Kolloide nach einem der Ansprüche 1 bis 7.

9. Topische Zusammensetzung, zu nicht therapeutischen Zwecken, umfassend die Kolloide nach einem der Ansprüche 1 bis 7.

10. Topische Zusammensetzung, nach Anspruch 8 oder 9, **dadurch gekennzeichnet, dass** sie keine hydrophilen und/ oder lipophilen, mineralischen und/ oder organischen Sonnenschutzfilter enthält.

11. Topische Zusammensetzung nach einem der Ansprüche 8 bis 10, **dadurch gekennzeichnet, dass** sie eine wässrige, mizelläre oder Pickering - Form hat.

12. Nahrungsergänzung mit Kolloiden nach einem der Ansprüche 1 bis 7.

**Claims**

1. Semiconductor colloids grafted covalently with an antioxidant **characterized in that** the colloids are semiconductor colloids constituted by at least one element chosen from the group comprising Cd, Zn, Cu, Ti, Mn, Fe, Ni, Cr, Hg, and combinations thereof and the antioxidant is a phenolic aldehyde or a phenolic acid.

2. The colloids according to claim 1, **characterized in that** the semiconductor colloids are colloids of zinc oxide, ZnO, or colloids of rutile titanium dioxide, $TiO_2$.

3. The colloids according to the preceding claims, **characterized in that** the antioxidant is chosen from the group comprising 2-hydroxybenzaldehyde, 3-hydroxybenzaldehyde, 4-hydroxybenzaldehyde, and 3,4-dihydroxybenzaldehyde.

4. The colloids according to claim 3, **characterized in that** the antioxidant is 3-hydroxybenzaldehyde.

5. The colloids according to claim 3, **characterized in that** the antioxidant is 3,4 dihydroxybenzaldehyde.

6. The colloids according to any of the preceding claims, **characterized in that** the covalent grafting is in the form of a spacer arm positioned between the semiconductor colloid and the antioxidant, the spacer arm comprising from 1 to 8 carbon atoms, preferably from 2 to 4 carbon atoms and having an alkoxysilane function capable of binding itself covalently to the colloid and a function of the hydroxyl type, of the phosphate type, or of the amine type capable of binding itself to the antioxidant.

7. The colloids according to the preceding claim, **characterized in that** the spacer arm is 3-(Aminopropyl)triethoxysilane.

8. A topical composition comprising the colloids according to any of claims 1 to 7.

9. Non-therapeutical topical composition comprising the colloids according to any of claims 1 to 7.

10. A topical composition according to claim 8 or 9, **characterized in that** it is devoid of any sun filters that are hydrophilic and/or lipophilic, inorganic and/or organic.

11. A topical composition according to any of claims 8 to 10, **characterized in that** it is in aqueous form, micellar form, or Pickering emulsion form.

12. A food supplement comprising colloids according to any of claims 1 to 7.

**Figure 1**

**Figure 2**

**Figure 3**

**Figure 4**

**Figure 5**

B

**Figure 6**

## RÉFÉRENCES CITÉES DANS LA DESCRIPTION

**Documents brevets cités dans la description**

- US 2003049287 A1